# FASCICULE DE BREVET EUROPEEN

(11) **EP 2 005 977 B1**
(45) Date de publication et mention de la délivrance du brevet: **16.12.2009**
(21) Numéro de dépôt: 08104444.8
(22) Date de dépôt: 17.06.2008
(51) Int. Cl.: A61L 27/22, A61L 27/24, A61L 27/50

(54) **Traitement de dispositifs médicaux implantables résistants à la calcification**
Behandlung von medizinischen Implantaten, die kalkbeständig sind
Treatment of implantable medical devices which are resistant to calcification.

(30) Priorité: 20.06.2007 FR 0755890
(43) Date de publication de la demande: 24.12.2008
(73) Titulaire: Laboratoires Perouse, 60173 Ivry Le Temple (FR)
(72) Inventeur: Shen, Ming, 92240 Malakoff (FR)
(74) Mandataire: Blot, Philippe Robert Emile

(56) Documents cités:
- EP-A- 0 174 737
- WO-A-99/22779
- US-A- 5 645 587
- US-A1- 2006 047 343
- SCHOEN F J ET AL: "Calcification of Tissue Heart Valve Substitutes: Progress Toward Understanding and Prevention" THE ANNALS OF THORACIC SURGERY, vol. 79, no. 3, mars 2005 (2005-03), pages 1072-1080, XP004762000 ISSN: 0003-4975
- DATABASE CA [Online] CHEMICAL ABSTRACTS SERVICE, COLUMBUS, OHIO, US; ARONOVA, G. A. ET AL: "Nature of active centers on the surface of silica determining its fibrinogenic character" XP002468616 extrait de STN Database accession no. 1969:499839 -& ARONOVA, G. A. ET AL: "Nature of active centers on the surface of silica determining its fibrinogenic character" GIGIENA TRUDA I PROFESSIONAL'NYE ZABOLEVANIYA, vol. 13, no. 5, 1969, pages 4-8, XP009095888 ISSN: 0016-9919

## Description

La présente invention a trait à des dispositifs médicaux implantables (désignés ci-après par le terme générique « d'implant médical »). Plus précisément, l'invention concerne des implants médicaux à base de protéines, notamment à base de collagène, rendus biocompatibles et résistants à la calcification, et plus spécifiquement un procédé de préparation de tels implants.

Il existe différents types d'implants médicaux, parmi lesquels les implants à base de protéines qui se révèlent particulièrement intéressants. Ces implants sont notamment plus avantageux que les implants à base de matériaux non biologiques, dans la mesure où ils permettent d'éviter, entre autres, des thromboses lorsqu'ils sont introduits dans un organisme vivant, notamment chez l'homme. Néanmoins, pour obtenir une telle biocompatibilité, les implants à base de protéines nécessitent un prétraitement.

En effet, les protéines présentes dans les implants à base de protéines sont généralement des protéines fibreuses (typiquement des collagènes) qui comportent des fonctions amines libres. Ces fonctions amines libres sont en partie responsables du rejet de l'implant dans un corps vivant, compte tenu de la reconnaissance par le système immunitaire de ces fonctions amines libres de la protéine.

Pour éviter ce phénomène de rejet, il est connu de traiter l'implant par un composé aldéhyde difonctionnel, typiquement le glutaraldéhyde.

Ce composé aldéhyde difonctionnel vise notamment à masquer les fonctions amines. Dans ce cadre, les fonctions aldéhydes réagissent avec les fonctions amines de l'implant en formant des fonctions imines (-C=N-). En outre, l'emploi d'un composé difonctionnel, comportant deux fonctions aldéhydes, permet de réaliser des pontages entre les différentes fibres de protéines.

Néanmoins, les implants à base de protéines traités par des composés de type glutaraldéhyde conduisent en général à une calcification assez rapide de l'implant lorsque ce dernier est disposé dans un corps vivant.

La calcification entraîne un durcissement de l'implant dû à l'accumulation de sels de calcium au niveau de l'implant. Ce phénomène nuit aux propriétés de l'implant, notamment dans l'utilisation de prothèses cardiaques ou vasculaires, ce qui nécessite un remplacement périodique de l'implant par voie chirurgicale. Pour plus de détails à ce sujet, on peut notamment se reporter à US 5,645,587.

Il a été proposé de post-traiter les implants traités par du glutaraldéhyde par différents composés, notamment par de l'acide oléique. Ces types de traitement visent en fait surtout à éliminer la présence de sous produits toxiques et ne permettent pas de limiter la calcification de façon suffisante.

Un but de la présente invention est de fournir des implants biocompatibles à base de protéines, dans lesquels les phénomènes de calcification sont limités par rapport à ceux observés avec des implants à base de protéines traités actuellement connus, de préférence en une mesure suffisante pour éviter le remplacement périodique de l'implant.

A cet effet, selon un premier aspect, l'invention a pour objet un procédé de traitement d'un implant comportant un substrat à base de protéines, ledit procédé comprenant les étapes où :
(A)- on traite le substrat à base de protéines par un composé porteur d'au moins un groupement aldéhyde, de préférence par un composé porteur d'au moins deux groupements aldéhydes, puis
(B)- on traite le substrat par un composé comprenant un borohydrure, puis
(C)- on traite le substrat issu de l'étape (B) par un dérivé porteur d'un groupe silane.

Par « implant », on entend, au sens de la présente description, un dispositif destiné à être implanté dans un corps vivant comprenant ou étant constitué par un substrat à base de protéines. Typiquement, l'implant traité selon l'invention est un implant cardiaque, notamment un implant valvulaire cardiaque.

Par « substrat à base de protéines », on entend ici un substrat comprenant une ou plusieurs protéines, généralement à titre de constituant majoritaire, par exemple en une teneur allant de 50 à 100% en masse. Ce substrat à base de protéines est constitutif de tout ou partie de l'implant. Le plus souvent, l'implant est entièrement constitué par ce substrat à base de protéines.

Le substrat peut également recouvrir d'autres matériaux tels que des prothèses, conduits, appareils chirurgicaux en contact avec le milieu vivant.

Dans le procédé de l'invention, le substrat à base de protéines de l'implant est soumis à un traitement de modification, qui assure notamment sa biocompatibilité. Par le terme « biocompatibilité » de l'implant, on entend ici que, lorsque l'implant est placé dans un corps vivant, et notamment chez l'homme, cet implant n'est pas reconnu par le système immunitaire, ce qui permet d'éviter des phénomènes de rejet des implants à base de protéines.

Les inventeurs ont maintenant mis en évidence que la succession des étapes (A), (B) et (C) permet de conférer à un implant à base de protéines une biocompatibilité élevée, tout en limitant l'apparition du phénomène de calcification de ce dernier.

En particulier, les travaux des inventeurs ont permis d'établir que la combinaison des étapes (B) et (C) permet de réduire fortement les phénomènes de calcification qui sont observés avec les implants actuellement connus, qui correspondent à des implants traités seulement selon l'étape (A) de la présente invention.

La limitation du phénomène de la calcification semble en partie s'expliquer par le fait que la succession des étapes (B) et (C) permet de réduire les fonctions aldéhydes libres introduites dans l'étape (A) en des fonctions alcools (dans de l'étape (B)), ces fonctions alcools étant protégées sous forme de fonctions siloxanes (dans l'étape (C)). Ces fonctions siloxanes présentent l'avantage d'être des fonctions stables, biocompatibles et peu propices à l'accumulation de sels de calcium.

Parallèlement, il s'avère en outre que l'étape (B) de réduction, conduit en plus de l'effet précité à une réduction d'autres fonctions introduites dans l'étape (A). Plus précisément, l'étape (B) conduit à la réduction de fonctions imines issues du couplage des amines libres du substrat de l'implant avec les aldéhydes difonctionnels de l'étape (A). Cette réaction des fonctions imines s'avère particulièrement avantageuse puisqu'il a été mis en évidence que ces fonctions imines favorisent également la calcification. L'étape (B) permet donc de supprimer ces groupements imines capables d'induire la calcification en les convertissant en des fonctions amines substituées, qui présentent l'avantage d'être stables.

Ainsi, le procédé de l'invention permet de limiter le phénomène de calcification en inhibant la présence de deux sources responsables de la calcification de l'implant. Par conséquent, l'implant préparé selon le procédé de l'invention présente l'avantage d'avoir un faible taux de calcification, ce qui permet, dans certains cas, d'éviter le remplacement de l'implant par le biais d'une intervention chirurgicale, ou tout du moins d'espacer dans le temps les interventions chirurgicales nécessaires au remplacement de l'implant.

Le plus souvent, le substrat à base de protéines présent dans l'implant traité selon l'invention comprend ou est constitué de protéines fibreuses. De préférence, le substrat est à base de collagène, d'élastine, de fibrine, de fibrinogène et/ou de protéoglycane.

L'implant traité selon l'invention est typiquement un implant valvulaire cardiaque, incluant tout ou partie d'une valve aortique et/ou d'un péricarde d'origine bovine, porcine, ovine, équine ou d'autruche.

Compte tenu de la présence de protéines, le substrat de l'implant comprend intrinsèquement des fonctions amines libres qui seraient responsables, au moins en partie, de phénomènes de rejet si le substrat non traité était implanté sans traitement dans un corps vivant.

Dans l'étape (A) du procédé selon l'invention, on traite le substrat à base de protéines constitutif de l'implant par un composé porteur d'au moins un groupement aldéhyde. Par souci de concision, ce composé sera désigné ci-après par le terme générique de « composé aldéhyde ». Le groupement aldéhyde de l'étape (A) est susceptible de réagir sur les fonctions amines libres du substrat en transformant ces dernières en fonctions imines. De façon générale, le composé porteur d'au moins une fonction aldéhyde est un composé représenté par la formule (I) suivante :

R-CHO (formule I)

où R est une chaîne hydrocarbonée comportant typiquement de 2 à 18 atomes de carbone, par exemple de 3 à 8 atomes de carbone éventuellement substitués par un hétéroatome tel qu'un atome de chlore, de fluor, de brome, d'azote, de phosphore ou de soufre. Ce groupement R peut éventuellement être substitué par un ou plusieurs autres groupes aldéhydes -CHO.

Avantageusement, le composé aldéhyde de l'étape (A) est hydrosoluble.

Selon un mode de réalisation avantageux, le composé aldéhyde employé dans l'étape (A) est un composé porteur d'au moins deux groupes aldéhydes -CHO, ce qui permet d'obtenir un pontage entre certaines des fibres protéiniques du substrat de l'implant traité selon le procédé de l'invention. Dans ce cadre, le composé aldéhyde de l'étape (A) peut notamment être de formule générale (II) : HOC-R²-CHO où R² est une chaîne hydrocarbonée comportant typiquement de 2 à 18 atomes de carbone, par exemple de 3 à 8 atomes de carbone éventuellement substitués par un hétéroatome tel qu'un atome de chlore, de fluor, de brome, d'azote, de phosphore ou de soufre. De préférence, le composé aldéhyde de l'étape (A) est le glutaraldéhyde.

Selon un mode de réalisation intéressant de l'étape (A), le substrat de l'implant est immergé dans une solution (S_{A}), notamment aqueuse, contenant le composé aldéhyde pendant une durée d'au moins deux semaines, de préférence d'au moins 1 mois. La solution (S_{A}) utilisée selon ce mode de réalisation peut notamment être préparée en diluant le composé aldéhyde dans un tampon, le pH de la solution (S_{A}) étant, de préférence, compris entre 5 et 9. A titre d'exemple, lorsque le composé aldéhyde est le glutaraldéhyde, le pH de la solution (S_{A}) est de l'ordre de 6 à 8. On peut notamment utiliser à titre de tampon une solution aqueuse de phosphate de sodium ou de potassium ou d'HEPES (acide 4-(2-hydroxyéthyl)-1-pipérazine éthane sulfonique), à une concentration comprise, de préférence, entre 10 et 50 mmol.l⁻¹, par exemple de l'ordre de 20 mmol.l⁻¹. La concentration en composé aldéhyde dans la solution (S_{A}) est typiquement comprise entre 0,1 et 1 %, par exemple de l'ordre de 0,6 à 0,7% en poids par rapport au volume total de la solution (S_{A}). Par ailleurs, on utilisera, de préférence, une solution (S_{A}) comprenant le composé aldéhyde avec une osmolarité (correspondant au nombre de moles de soluté par kilogramme de solvant) comprise entre 200 et 400 mOsMol.l⁻¹, par exemple de l'ordre de 300 mOsMol.l⁻¹.

Le traitement de l'étape (A) est préférentiellement effectué sous agitation. Par ailleurs, la température du milieu réactionnel de l'étape (A) est, de préférence, comprise entre 10 et 70°C. Par exemple, l'étape (A) peut être conduite à température ambiante, typiquement entre 20 et 30°C, notamment aux alentours de 25°C.

Le traitement de l'étape (A) conduit le plus souvent à une réaction entre les fonctions amines libres et le composé aldéhyde, selon la réaction schématique suivante :

[substrat]-NH₂ + R-CHO → [substrat]-N=CH-R

où R a la signification précitée. Le plus souvent, et en particulier, lorsqu'on emploie les solutions préférentielles précitées, l'étape (A) conduit à une réaction de la majeure partie voire de la totalité des fonctions amines libres initialement présentes sur le substrat de l'implant.

Dans le cas de l'emploi d'un composé porteur d'au moins deux fonctions aldéhydes, l'étape (A) conduit en outre à des réactions induisant un pontage entre certaines fibres du substrat, selon la réaction schématique suivante :

2 [substrat]-NH₂ + OHC-R²-CHO → [substrat]-N=CH-R²-CH=N-[substrat]

où R² est tel que décrit ci-dessus.

Dans le cas de composés aldéhydes difonctionnels, certains de ces composés aldéhydes peuvent voir une de leurs fonctions aldéhydes restée libre selon la réaction suivante :

[substrat]-NH₂ + OHC-R²-CHO→ [substrat]-N=CH-R²-CHO

où R² est tel que décrit ci-dessus.

Ainsi, à l'issue de l'étape (A), il reste des groupements aldéhydes -CHO libres, qui sont susceptibles d'induire des phénomènes de calcification. En outre, le substrat est porteur de fonctions imines (-N=C-) également sources de calcification.

C'est ce type de réaction qui est observé lorsqu'on traite des implants à base de collagène par du glutaraldéhyde selon les procédés connus de l'état de la technique.

Un but des étapes (B) et (C) est de supprimer sensiblement tous ces groupements aldéhydes -CHO libres et les fonctions imines introduits à l'issue de l'étape (A).

Dans l'étape (B) du procédé selon l'invention, on traite le substrat par un composé comprenant un borohydrure, ce qui permet de réduire à la fois les fonctions aldéhydes en des fonctions alcools et les fonctions imines en des fonctions amines de manière sélective de sorte à ne pas modifier les fonctions amides constitutives des protéines.

Le composé comprenant un borohydrure qui est employé dans l'étape (B) est de préférence un dérivé d'un métal alcalin tel que le sodium, le lithium, le potassium. De préférence, le composé comprenant un borohydrure est un cyanoborohydrure métallique, tel que le cyanoborohydrure de sodium, par exemple.

Selon un mode de réalisation intéressant, l'étape (B) peut être conduite en immergeant partiellement ou totalement le substrat issu de l'étape (A) du procédé selon l'invention dans une solution (S_{B}) du composé comprenant un borohydrure, pendant un durée typiquement d'au moins 1 h, généralement d'au moins 5h, par exemple pendant 10 à 30h, typiquement de l'ordre de 24h. La solution (S_{B}) utilisée selon le mode de réalisation peut notamment être obtenue en diluant le composé comportant un borohydrure dans une solution tampon. La solution (S_{B}) a typiquement un pH compris entre 5 et 11. Un tampon adapté est notamment une solution aqueuse comportant du phosphate de sodium disodique dont la concentration est typiquement comprise 20 et 500 mmol.l⁻¹, par exemple de l'ordre de 200 mmol.l⁻¹. La concentration du composé comprenant un borohydrure est notamment comprise entre 10 et 160 mmol.l⁻¹, mieux égale à environ 80 mmol.l⁻¹.

Le traitement de l'étape (B) est généralement effectué sous agitation, par exemple de l'ordre de 50 tours.min⁻¹. La température du milieu réactionnel de l'étape (B) est, de préférence, comprise entre 10 et 70°C. Par exemple, l'étape (B) peut être conduite à température ambiante, par exemple entre 20 et 30°C typiquement aux alentours de 25°C.

Typiquement, la réaction qui a lieu dans l'étape (B) est la suivante : 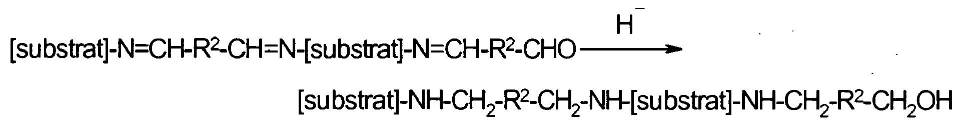 où R² est tel que décrit ci-dessus.

Ainsi, à l'issue de l'étape (B), le substrat comporte des fonctions alcools terminales pouvant réagir avec des enzymes de phosphorylation, ce qui est notamment susceptible de provoquer une dégradation de l'implant. En effet, les groupements phosphates de ces enzymes se lient facilement aux cations de calcium en amorçant la calcification du substrat. A terme, une telle dégradation de l'implant nécessiterait également le remplacement de ce dernier par voie chirurgicale.

Un but de l'étape (C) est de s'affranchir de la présence des fonctions alcools terminales introduites dans l'étape (B). A cet effet, dans l'étape (C) du procédé de l'invention, on traite le substrat issu de l'étape (B) par un dérivé porteur d'un groupe silane afin de convertir les fonctions alcools en fonctions siloxanes. Les fonctions siloxanes ainsi formées sont non réactives, et elles sont en outre définitives, au sens où la réaction de protection des fonctions alcools en fonctions siloxanes est irréversible (la déprotection impliquerait une destruction du substrat). Cette protection définitive des fonctions alcools terminales permet d'éviter à l'implant toute dégradation par des composés actifs issus d'un organisme vivant. De plus, les fonctions siloxanes ne sont pas reconnues par le système immunitaire et elles ne favorisent pas le phénomène de calcification.

En règle générale, le dérivé porteur d'un groupe silane employé dans l'étape (C) comporte un groupe électroattracteur lié directement à l'atome de silicium. Ce groupement électroattracteur est typiquement choisi parmi les halogènes, les groupes hétéroaryles comportant de 5 à 15 atomes de carbone et, éventuellement, 2 ou 3 hétéroatomes typiquement choisis parmi le groupe constitué par les halogènes, tels que le fluor, le chlore, le brome et l'iode, les pnictogènes correspondant aux éléments de la quinzième colonne de la classification périodique des éléments, tels que l'azote et le phosphore, et les chalcogènes correspondant aux éléments de la seizième colonne de la classification périodique, tels que l'oxygène et le soufre.

De préférence, le groupe électroattracteur présent dans le dérivé porteur d'un groupe silane employé dans l'étape (C) est un atome de chlore, un atome de brome, un groupe imidazole.

Selon une variante, le groupe électroattracteur peut être ceux précités connus par l'homme du métier.

D'une manière préférentielle, le dérivé porteur d'un groupe silane utilisé dans l'étape (C) est un trialkylsilylimidazole ou un halogénotrialkylsilane, par exemple le triméthylsilylimidazole ou le chlorotriméthylsilane (également désigné chlorométhylsilane ci-après).

De préférence, le dérivé porteur d'un groupe silane employé dans l'étape (C) est introduit sous la forme d'une solution (S_{C}) dans laquelle au moins un composé hydrosoluble, non toxique, choisi dans le groupe constitué par le tétrahydrofurane ou bien encore le dioxane, le diglyme, le triglyme, le diméthylformamide, le diméthylacétamide, la N-méthylpyrolidone est utilisé à titre de solvant. A titre d'exemple, le dérivé porteur d'un groupe silane peut être dilué dans le tétrahydrofurane selon un facteur de dilution compris entre 1 et 20%, mieux égal à environ 10 %.

Typiquement, dans l'étape (C), on traite le substrat issu de l'étape (B) dans la solution (S_{C}) précitée pendant une durée comprise entre 1 et 30 minutes, par exemple pendant 4 à 6 minutes, typiquement aux alentours de 5 min. Le traitement de l'étape (C) peut notamment être effectué sous agitation, par exemple aux alentours de 50 tours.min⁻¹. Par ailleurs, dans l'étape (C), la température du milieu réactionnel est de préférence, comprise entre 10 et 35°C. Par exemple, l'étape (C) peut être conduite à température ambiante, par exemple entre 20 et 30°C, typiquement aux alentours de 25°C.

Selon une variante avantageuse, le procédé selon l'invention comporte, en plus des étapes (A), (B) et (C) précitées, une étape (A1) intermédiaire, située entre les étapes (A) et (B), où le substrat tel qu'obtenu à l'issu de l'étape (A) est traité par un composé porteur d'au moins deux fonctions amines préalablement à la mise en oeuvre des étapes (B) et (C).

Selon cette variante du procédé de l'invention, une partie des fonctions aldéhydes libres formées à l'issue de l'étape (A) réagit sur des fonctions amines présentes sur les composés porteurs de fonctions amines employés dans l'étape (A1) formant ainsi des liaisons imines selon la réaction schématique suivante :

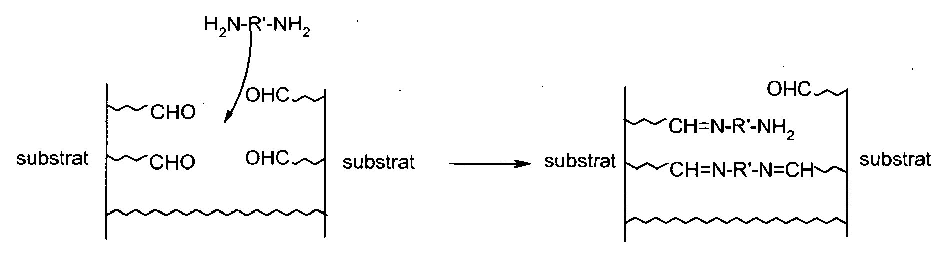

Comme l'illustre le schéma ci-dessus, l'étape (A1) conduit, entre autres avantages, à une augmentation du pontage entre les chaînes protéiniques du substrat.

Par ailleurs, l'étape (A1) masque une partie des fonctions aldéhydes -CHO laissées libres à l'issue de l'étape (A). Comme l'indique le schéma, ce masquage peut induire la formation de chaînes greffées porteuses de fonctions amines terminales libres. La présence de ces fonctions amines terminales libres n'est néanmoins pas gênante pour la biocompatibilité de l'implant. En effet, ces fonctions ne sont pas reconnues par le système immunitaire et n'entraînent donc pas de rejet.

De préférence, le composé porteur d'au moins deux fonctions amines employé dans l'étape (A1) est une diamine de formule NH₂-A-NH₂ où A représente une chaîne hydrocarbonée linéaire ou ramifiée comportant de 1 à 20 atomes de carbone éventuellement substitués par un ou plusieurs hétéroatomes choisis parmi le groupe constitué par les halogènes, tels que le fluor, le chlore, le brome et l'iode, les pnictogènes correspondant aux éléments de la colonne Vb de la classification périodique des éléments, tels que l'azote et le phosphore, et les chalcogènes correspondant aux éléments de la colonne Vlb de la classification périodique, tels que l'oxygène et le soufre. D'une manière encore plus préférentielle, le composé porteur d'au moins deux fonctions amines est le poly(propylèneglycol)bis(2-aminopropyléther), la lysine, la spermine ou la putrescine.

Selon un mode de réalisation, l'étape (A1) peut être conduite en immergeant partiellement ou totalement l'implant issu de l'étape (A) du procédé selon l'invention dans une solution aqueuse (S_{A1}) comprenant le composé porteur d'au moins une fonction amine, typiquement à une concentration comprise entre 10 et 200 mmol.l⁻¹, mieux encore entre 40 et 80 mmol.l⁻¹, par exemple à une concentration de l'ordre de 60 mmol.l⁻¹. Typiquement, l'étape (A1) est effectuée en immergeant partiellement ou totalement l'implant dans la solution (S_{A1}) pendant une durée typiquement comprise entre 10 et 100 min, par exemple pendant environ 60 min.

Le traitement de l'étape (A1) peut typiquement être effectué sous agitation, par exemple aux alentours de 50 tours.min⁻¹. La température du milieu réactionnel de l'étape (A1) est de préférence, comprise entre 10 et 70°C. Par exemple, l'étape (A1) peut être conduite à température ambiante, par exemple entre 20 et 30°C, typiquement aux alentours de 25°C.

Selon un mode de réalisation particulier, suite à l'étape (A) et à l'éventuelle étape (A1) et préalablement à l'étape (B), le procédé de l'invention peut comprendre en outre une étape de modification de pH, pour porter le milieu réactionnel à un pH notamment compris entre 5 et 9, mieux encore entre 5,5 et 6,5, par exemple égal à environ 6, ce qui favorise la réduction ultérieure de l'étape (B). Cette étape peut notamment être conduite en traitant le milieu issu de l'étape (A) et de l'éventuelle étape (A1) par l'acide morpholinoéthanesulphonique (MES) pendant une durée comprise entre 10 et 50 h, mieux encore entre 20 et 30 h, par exemple pendant environ 24h.

Selon un mode de réalisation, avant et après chaque étape d'un procédé de l'invention, l'implant peut être lavé par de l'eau, typiquement par de l'eau ultra pure. Par « eau ultra pure », on entend ici que la résistivité de l'eau est égale à environ 18,2 MΩ.cm à environ 25°C. Le lavage par de l'eau, notamment par de l'eau ultra pure, permet d'éliminer les réactifs excédentaires présents à l'issue de chacune des étapes du procédé, ce qui permet de rendre l'implant exempt de tout composé qui pourrait interagir avec l'organisme du corps vivant. A titre d'exemple, l'implant peut être rincé au moins deux fois, mieux trois fois, à l'eau ultra pure avant et après chacune des étapes (A), (B) et (C) (et (A1), le cas échéant.

Selon un mode de réalisation, le milieu de l'étape (A) peut être isolé afin de conserver l'implant pour la mise en oeuvre ultérieure des étapes (B) et (C) ou des étapes (A1), (B) et (C).

Selon un mode de réalisation, l'implant traité selon les étapes (A), éventuellement (A1), (B) et (C) peut subir un autre traitement à la suite de l'étape (C). Ce traitement supplémentaire peut être destiné à améliorer encore davantage la résistance de l'implant face à la calcification. A titre d'exemple, l'implant issu de l'étape (C) peut être traité par les solutions anticalcifiantes actuellement connues, telles que le « sterilent » (22% d'éthanol, 4% de formaldéhyde et 1,2% de Tween 80 (polysorbate 80) dans le reste d'eau, les pourcentages étant donnés en volume par rapport au volume total de la solution.

Quelque soit le mode de mise en oeuvre du procédé de l'invention, on obtient à l'issue de ce procédé un implant qui n'est sensiblement plus porteur de fonctions propres à induire une calcification.

Selon un deuxième aspect, l'invention a également pour objet l'implant à base de protéines traité susceptible d'être obtenu à l'issue du procédé de l'invention.

Un implant traité selon l'invention est en général sensiblement exempt de fonctions aldéhydes -CHO libres et de fonctions imines.

Par ailleurs, l'implant traité selon l'invention est porteur de fonctions siloxanes terminales.

Par fonctions siloxanes terminales, on entend des chaînes hydrocarbonées comportant des hétéroatomes, tels qu'un atome d'azote, d'oxygène, de chlore, de brome, d'iode, de phosphore, interrompues par un atome de silicium en surface de l'implant.

Ainsi, l'implant selon l'invention présente l'avantage d'avoir une faible calcification.

De préférence, l'implant traité selon l'invention est un implant valvulaire cardiaque.

Différents aspects et avantages de l'invention ressortent encore à la lecture des exemples non limitatifs suivants.

### EXEMPLES

### Substrat utilisé

Pour la préparation de l'implant, on a utilisé à titre de substrat un morceau d'un péricarde bovin.

### Préparation des solutions.

Dans les exemples qui suivent, l'eau ultra pure utilisée est une solution aqueuse ayant une résistivité égale à environ 18,2 MΩ à environ 25°C.

### Solution (S1) de glutaraldéhyde

Environ 6,25 g de glutaraldéhyde a été dilué dans environ 1 l d'une solution tampon comportant du phosphate de sodium et du phosphate de potassium à environ 20mmol.l⁻¹. On a ainsi obtenu une concentration finale en glutaraldéhyde d'environ 0,625% en poids par rapport au volume total de la solution (S1).

L'osmolarité de la solution était égale à environ 300mOsmol.l⁻¹ en ajoutant environ 5,3 g de chlorure de sodium dans la solution (51).

Le pH de la solution (S1) était d'environ 7,4.

### Solution (S2) de poly(propylèneglycol)bis(2-aminopropyléther) (Jeffamine)

Environ 1,437 ml de poly(propylèneglycol)bis(2-aminopropyléther) ont été dilués dans environ 98,563 ml d'eau ultra pure.

La concentration en poly(propylèneglycol)bis(2-aminopropyléther) dans la solution (S2) était égale à environ 60mmol.l⁻¹.

### Solution (S3) de cyanoborohydrure de sodium (NaCNBH₃)

Environ 0,5g de cyanoborohydrure de sodium a été dissous dans environ 10 ml d'eau ultra pure pendant une nuit. La solution obtenue a été ensuite diluée dans environ 90ml d'une autre solution comportant 200 mmol.l⁻¹ de Na₂HPO₄.

La concentration finale en cyanoborohydrure de sodium dans la solution (S3) était d'environ 80 mmol.l⁻¹.

### Solution (S4) de chlorométhylsilane (chlorotriméthylsilane)

Environ 10 ml de chlorométhylsilane ont été dilués dans environ 90ml de trétrahydrofurane.

### Solution (S5) de triméthylsilylimidazole

Environ 10 ml de triméthylsilylimidazole ont été dilués dans environ 90ml de tétrahydrofurane.

### Solution (S6) de « stérilent »

Environ 220 ml d'éthanol absolu, 108 ml de formaldéhyde à 37% et 12 ml de Tween 80 ont été dilués dans environ 660 ml de tampon de phosphate de sodium et de potassium. La concentration finale de phosphate était d'environ 20 mmol.l⁻¹ et le pH de la solution était d'environ 7,4.

### Exemple 1 : traitement de substrat selon les étapes (A), (B) et (C) du procédé de l'invention

### • Traitement par la solution (S1) de glutaraldéhyde

Le substrat a été traité par la solution (S1) au moins un mois à température ambiante (environ 25°C).

A l'issue de ce traitement, le substrat traité a été coupé en carrés de 8 mm d'environ 7 mm de côté.

Les échantillons issus du substrat ainsi traité ont été rincés trois fois à l'eau ultra pure.

### • Traitement par la solution (S3) de cyanoborohydrure de sodium

Ces échantillons rincés ont été introduits dans une bouteille à section rectangulaire de 150ml contenant environ 100ml de la solution (S3). Le milieu réactionnel a été ensuite laissé sous agitation à environ 50 tours.min⁻¹ à température ambiante (environ 25°C) pendant environ 24 heures.

Les échantillons ainsi traités ont été rincés trois fois à l'eau ultra pure.

### • Traitement par la solution (S4) de chlorométhylsilane

Les échantillons ainsi rincés ont été introduits dans une bouteille à section rectangulaire de 150ml contenant 100ml de la solution de chlorométhylsilane (S4). Le milieu réactionnel a été ensuite laissé sous une agitation égale à environ 50 tours.min⁻¹ à température ambiante (environ 25°C) pendant environ 5 minutes.

Les échantillons ainsi traités ont été rincés trois fois à l'eau ultra pure.

### Exemple 2 : traitement du substrat selon les étapes (A), (A1), (B) et (C) du procédé de l'invention

### • Traitement par la solution (S1) de glutaraldéhyde

Le substrat a été traité par la solution (S1) au moins un mois à température ambiante (environ 25°C).

A l'issue de ce traitement, le substrat traité a été coupé en carrés d'environ 7 mm de côté.

Les échantillons issus du substrat ainsi traité ont été rincés trois fois à l'eau ultra pure.

### • Traitement par la solution (S2) de poly(propylènealycol)bis(2-aminopropyléther) (Jeffamines)

Les échantillons ainsi rincés ont été introduits dans une bouteille à section rectangulaire de 150ml contenant 100ml de la solution (S2). La bouteille a été mise sous agitation à environ 50 tours.min⁻¹ pendant environ 1 heure à température ambiante (environ 25°C).

Environ 5,76 g d'acide morpholinoéthanesulphonique (MES) ont été ajoutés au milieu réactionnel. Le milieu réactionnel a été laissé sous agitation à environ 50 tours.min⁻¹ à température ambiante (environ 25°C) pendant environ 23 heures.

Le pH final du milieu réactionnel était d'environ 6.

Les échantillons ainsi traités ont été rincés trois fois à l'eau ultra pure.

### • Traitement par la solution (S3) de cyanoborohydrure de sodium

Ces échantillons rincés ont été introduits dans une bouteille à section rectangulaire de 150ml contenant environ 100ml de la solution (S3). Le milieu réactionnel a été ensuite laissé sous agitation à environ 50 tours.min⁻¹ à température ambiante (environ 25°C) pendant environ 24 heures.

Les échantillons ainsi traités ont été rincés trois fois à l'eau ultra pure.

### • Traitement par la solution (S4) de chlorométhylsilane

Les échantillons ainsi rincés ont été introduits dans une bouteille à section rectangulaire de 150m1 contenant 100ml de la solution de chlorométhylsilane (S4). Le milieu réactionnel a été ensuite laissé sous une agitation égale à environ 50 tours.min⁻¹ à température ambiante (environ 25°C) pendant environ 5 minutes.

Les échantillons ainsi traités ont été rincés trois fois à l'eau ultra pure.

### Exemple 3 : traitement du substrat selon les étapes (A), (A1), (B) et (C) du procédé de l'invention

On a répété le même protocole opératoire excepté que l'on a utilisé dans la dernière étape (C) la solution de triméthylsilylimidazole (S5) au lieu de la solution de chlorométhylsilane (S4)

### Exemple 4 : traitement du substrat selon les étapes (A), (A1), (B) puis (C) du procédé de l'invention suivie d'une étape ultérieure de traitement par une solution anticalcifiante (sterilent).

On a repris le protocole opératoire de l'exemple 3, à l'issue duquel on a mis en oeuvre l'étape suivante.

Les échantillons ainsi traités ont été introduits dans une bouteille à section rectangulaire de 150ml contenant 100ml de la solution (S6). Le milieu réactionnel a été ensuite laissé sous une agitation égale à environ 50 tours.min⁻¹ à température ambiante (environ 25°C) pendant environ 24 heures.

Les échantillons ainsi traités ont été rincés trois fois à l'eau ultra pure.

### Exemples comparatifs

### Exemple comparatif 1 : traitement du substrat selon l'étape (A) uniquement

Le substrat a été traité par la solution (S1) de glutaraldéhyde au moins un mois à température ambiante (environ 25°C).

A l'issue de ce traitement, le substrat traité a été coupé en carrés de 7 mm de côté.

Les échantillons issus du substrat ainsi traité ont été rincés trois fois à l'eau ultra pure puis ont été conservé dans la solution (S1) jusqu'à l'implantation chez les rats.

### Exemple comparatif 2 : traitement du substrat selon l'étape (A) suivie d'une étape de traitement par la solution (S6) de stérilente

On a repris le même protocole opératoire que l'exemple comparatif 1, à l'issue duquel on a mis en oeuvre l'étape suivante.

Les échantillons ainsi traités par la solution (S1) de glutaraldéhyde ont été introduits dans une bouteille à section rectangulaire de 150ml contenant une solution aqueuse (S6) de 100ml. Le milieu réactionnel a été ensuite laissé sous une agitation égale à environ 50 tours.min⁻¹ à environ 32°C pendant environ 9 heures.

Les échantillons ainsi traités ont été rincés trois fois à l'eau ultra pure.

### Exemple comparatif 3 : traitement du substrat selon l'étape (A) suivie d'une étape de traitement par du tétrahydrofurane.

On a repris le même protocole opératoire que l'exemple comparatif 1, à l'issue duquel on a mis en oeuvre l'étape suivante.

Les échantillons ainsi traités par la solution (S1) de glutaraldéhyde ont été introduits dans une bouteille à section rectangulaire de 150ml contenant 100ml de tétrahydrofurane. Le milieu réactionnel a été ensuite laissé sous une agitation égale à environ 50 tours.min⁻¹ à température ambiante (environ 25°C) pendant environ 24 heures.

Les échantillons ainsi traités ont été rincés trois fois à l'eau ultra pure.

### Exemple comparatif 4 : traitement du substrat selon l'étape (A) suivie de l'étape (C) en utilisant la solution (S4)

On a repris le même protocole opératoire que l'exemple comparatif 1, à l'issue duquel on a mis en oeuvre l'étape suivante.

Les échantillons ainsi traités par la solution (S1) de glutaraldéhyde ont été introduits dans une bouteille de section rectangulaire de 150ml contenant 100ml de la solution de chlorométhylsilane (S4). Le milieu réactionnel a été ensuite laissé sous une agitation égale à environ 50 tours.min⁻¹ à température ambiante (environ 25°C) pendant environ 5 minutes.

Les échantillons ainsi traités ont été rincés trois fois à l'eau ultra pure.

### Exemple comparatif 5 : traitement du substrat selon l'étape (A) suivie de l'étape (C) en utilisant la solution (S5)

On a repris le protocole opératoire de l'exemple comparatif 4 en remplaçant la solution (S4) de chlorométhylsilane par la solution (S5) de triméthylsilylimidazole.

### Exemple comparatif 6 : traitement du substrat selon les étapes (A) puis (C) en utilisant la solution (S5) suivie d'une étape de traitement par la solution (S6) de stérilente.

On a repris le protocole de l'exemple comparatif 5, à l'issue duquel on a mis en oeuvre l'étape suivante.

Les échantillons ainsi traités par la solution (S1) de glutaraldéhyde ont été introduits dans une bouteille à section rectangulaire de 150ml contenant 100ml de la solution (S6). Le milieu réactionnel a été ensuite laissé sous une agitation égale à environ 50 tours.min⁻¹ à température ambiante (environ 25°C) pendant environ 24 heures.

Les échantillons ainsi traités ont été rincés trois fois à l'eau ultra pure.

### Exemple comparatif 7 : traitement du substrat selon les étapes (A), (A1) puis (C) en utilisant la solution (S4).

On a repris le protocole opératoire de l'exemple comparatif 1, à l'issue duquel on a mis oeuvre les étapes suivantes.

### • Traitement par la solution (S2) de poly(propylèneglycol)bis(2-aminopropyléther)

Les échantillons ainsi traités ont été introduits dans une bouteille à section rectangulaire de 150ml contenant 100ml de la solution (S2). La bouteille a été mise sous agitation à environ 50 tours.min⁻¹ pendant environ 1 heure à température ambiante (environ 25°C).

Environ 5,76 g d'acide morpholinoéthanesulphonique (MES) ont été ajoutés au milieu réactionnel. Le milieu réactionnel a été laissé sous agitation à environ 50 tours.min⁻¹ à température ambiante (environ 25°C) pendant environ 23 heures.

Le pH final du milieu réactionnel était d'environ 6.

Les échantillons ainsi traités ont été rincés trois fois à l'eau ultra pure.

### • Traitement par la solution (S4) de chlorométhylsilane

Les échantillons ainsi rincés ont été introduits dans une bouteille à section rectangulaire de 150ml contenant 100ml de la solution de chlorométhylsilane (S4). Le milieu réactionnel a été ensuite laissé sous une agitation égale à environ 50 tours.min⁻¹ à température ambiante (environ 25°C) pendant environ 5 minutes.

Les échantillons ainsi traités ont été rincés trois fois à l'eau ultra pure.

### Etude de la calcification chez le rat

Les échantillons non traités ou traités issus des exemples décrits ci-dessus ont été implantés en sous-cutané chez des rats nouveau-nés âgés de 12 jours.

Les rats ont été sevrés après 9 jours d'implantation et soumis au régime consistant en un apport de granules contenant environ 332 mg de calcium, environ 236 mg de phosphore, environ 9,6 mg de fer, environ 60 UI de Vitamine D3 par kg de rat et un apport hydrique libre.

Après dix mois d'implantation, les rats ont été sacrifiés et les échantillons ont été explantés pour être analysés.

Les échantillons ont été ensuite nettoyés à l'eau ultra pure, lyophilisés puis pesés (en poids sec en mg). Les échantillons lyophilisés ont été digérés dans environ 1 ml d'acide nitrique à 70 % à environ 95°C pendant environ 15 min. Le volume du milieu a ensuite été complété à environ 5 ml d'eau ultra pure dans une fiole jaugée de 5 ml.

Le dosage de calcium de ces échantillons a été effectué par le spectrophotomètre atomique à flamme. Le calcium ainsi dosé provient essentiellement du phénomène de calcification de l'implant.

Les résultats du dosage de calcium issu des échantillons ayant subi ou non différents traitements sont résumés dans le tableau ci-après.

| **Disque traité par** | **Pourcentage de calcium par rapport au poids total du disque** |
|---|---|
| aucun traitement | 9,75 % |
| Exemple 1 | 0,67 % |
| Exemple 2 | 0,07 % |
| Exemple 3 | 0,56 % |
| Exemple 4 | 0,49 % |
| Exemple comparatif 1 | 20,82 % |
| Exemple comparatif 2 | 1,67 % |
| Exemple comparatif 3 | 9,10 % |
| Exemple comparatif 4 | 1,09 % |
| Exemple comparatif 5 | 1,15 % |
| Exemple comparatif 6 | 1,33 % |
| Exemple comparatif 7 | 1,14 % |

| | |
|---|---|
| Glut.=glutaraldéhyde | |

Selon les résultas indiqués dans le tableau ci-dessus, le substrat traité par le cyanoborohydrure de sodium suivie par le traitement par un dérivé porteur d'un groupe silane, notamment par le chlorométhylsilane ou le triméthylsilylimidazole, diminue nettement le phénomène de calcification par rapport à un traitement avec la solution commerciale, le sterilent.

De plus, si l'implant est traité en outre par un composé porteur d'au moins deux fonctions amines, le poly(propylèneglycol)bis(2-aminopropyléther), alors le phénomène de calcification est encore moins important.

## Revendications

1. Procédé de traitement d'un implant comportant un substrat à base de protéines, comprenant les étapes où :
(A)- on traite le substrat à base de protéines par un composé porteur d'au moins un groupement aldéhyde, puis
(B)- on traite le substrat par un composé comprenant un borohydrure, puis
(C)- on traite le substrat issu de l'étape (B) par un dérivé porteur d'un groupe silane.

2. Procédé selon la revendication 1, dans lequel le substrat à base de protéines est à base de collagène, d'élastine de fibrine, de fibrinogène et/ou de protéoglycane.

3. Procédé selon la revendication 1 ou 2, dans lequel l'implant est un implant valvulaire cardiaque, incluant tout ou partie d'une valve aortique et/ou d'un péricarde d'origine bovine, porcine ou ovine.

4. Procédé selon l'une quelconque des revendications précédentes, dans lequel on emploie dans l'étape (A) un composé porteur d'au moins deux groupes aldéhydes.

5. Procédé selon l'une quelconque des revendications précédentes, dans lequel le composé comprenant un borohydrure qui est employé dans l'étape (B) est un dérivé d'un métal alcalin.

6. Procédé selon la revendication 5, dans lequel le composé comprenant un borohydrure qui est employé dans l'étape (B) est le cyanoborohydrure de sodium.

7. Procédé selon l'une quelconque des revendications précédentes, dans lequel le dérivé porteur d'un groupe silane employé dans l'étape (C) comporte un groupe électroattracteur lié directement à l'atome de silicium et choisi parmi les halogènes, les groupes hétéroaryles comportant de 5 à 15 atomes de carbone et 2 ou 3 hétéroatomes choisis parmi le groupe constitué par les halogènes, les pnictogènes et les chalcogènes.

8. Procédé selon la revendication 7, dans lequel le groupe électroattracteur présent dans le dérivé porteur d'un groupe silane employé dans l'étape (C) est un atome de chlore, un atome de brome ou un groupe imidazole.

9. Procédé selon la revendication 8, dans lequel le dérivé porteur d'un groupe silane utilisé dans l'étape (C) est le triméthylsilylimidazole ou le chlorotriméthylsilane.

10. Procédé selon l'une quelconque des revendications précédentes, comportant une étape (A1) intermédiaire, située entre les étapes (A) et (B), où le substrat tel qu'obtenu à l'issu de l'étape (A) est traité par un composé porteur d'au moins deux fonctions amines préalablement à la mise en oeuvre des étapes (B) et (C).

11. Procédé selon la revendication 10, où le composé porteur d'au moins deux fonctions amines est une diamine de formule NH₂-A-NH₂ où A représente une chaîne hydrocarboné linéaire ou ramifiée comportant de 1 à 20 atomes de carbone éventuellement substitués par un ou plusieurs hétéroatomes choisis parmi le groupe constitué par les halogènes, les pnictogènes et les chalcogènes.

12. Procédé selon la revendication 11, dans lequel le composé porteur d'au moins deux fonctions amines est le poly(propylèneglycol)bis(2-aminopropyléther), la lysine, la spermine ou la putrescine.

13. Implant à base de protéine traité susceptible d'être obtenu à l'issu du procédé de traitement selon l'une quelconque des revendications précédentes.

14. Implant selon la revendication 13, où ledit implant est sensiblement exempt de fonctions aldéhydes -CHO libres et de fonctions imines.

15. Implant selon la revendication 13 ou 14, où ledit implant est un implant valvulaire cardiaque.

## Claims

1. A method for treating an implant comprising a protein-based substrate, comprising the steps in which:
(A) - the protein-based substrate is treated with a compound bearing at least one aldehyde group, then
(B) - the substrate is treated with a compound comprising a borohydride, then
(C) - the substrate obtained from step (B) is treated with a derivative bearing a silanol group.

2. A method according to claim 1, in which the protein-based substrate is based on collagen, elastin, fibrin, fibrinogen and/or proteoglycan.

3. A method according to claim 1 or claim 2, in which the implant is a cardiac valve implant, including all or part of an aortic valve and/or of a pericardium of bovine, porcine or ovine origin.

4. A method according to any one of the preceding claims, in which a compound bearing at least two aldehyde groups is used in step (A).

5. A method according to any one of the preceding claims, in which the compound comprising a borohydride used in step (B) is an alkali metal derivative.

6. A method according to claim 5, in which the compound comprising a borohydride used in step (B) is sodium cyanoborohydride.

7. A method according to any one of the preceding claims, in which the derivative bearing a silanol group used in step (C) comprises an electron-attracting group directly attached to the silicon atom and selected from among halogens, heteroaryl groups comprising from 5 to 15 carbon atoms and 2 or 3 heteroatoms selected from among the group made up of halogens, pnictogens and chalcogens.

8. A method according to claim 7, in which the electron-attracting group present in the derivative bearing a silanol group used in step (C) is a chlorine atom, a bromine atom or an imidazole group.

9. A method according to claim 8, in which the derivative bearing a silanol group used in step (C) is trimethylsilylimidazole or chlorotrimethylsilane.

10. A method according to any one of the preceding claims, comprising an intermediate step (A1), located between steps (A) and (B), in which the substrate as obtained on completion of step (A) is treated with a compound bearing at least two amine functions before steps (B) and (C) are carried out.

11. A method according to claim 10, in which the compound bearing at least two amine functions is a diamine of the formula NH₂-A-NH₂ in which A represents a linear or branched hydrocarbon chain comprising from 1 to 20 carbon atoms optionally substituted by one or more heteroatoms selected from among the group made up of halogens, pnictogens and chalcogens.

12. A method according to claim 11, in which the compound bearing at least two amine functions is poly(propylene glycol) bis(2-aminopropyl ether), lysine, spermine or putrescine.

13. A treated protein-based implant capable of being obtained on completion of the treatment method according to any one of the preceding claims.

14. An implant according to claim 13, in which said implant contains substantially no free aldehyde -CHO functions and imine functions.

15. An implant according to claim 13 or claim 14, in which said implant is a cardiac valve implant.

## Patentansprüche

1. Verfahren zur Behandlung eines Implantats, das ein Substrat umfasst, das auf Proteinen basiert, das die folgenden Schritte umfasst, worin:
(A)- Behandeln des Substrats, das auf Proteinen basiert, mit einer Verbindung, die mindestens eine Aldehydgruppe umfasst, dann
(B)- Behandeln des Substrats mit einer Verbindung, die Borhydrid umfasst, dann
(C)- Behandeln des Substrats, das aus Schritt (B) hervorgegangen ist, mit einem Derivat, das eine Silangruppe trägt.

2. Verfahren nach Anspruch 1, wobei das Substrat, das auf Proteinen basiert, auf Collagen, Elastin-Fibrin, Fibrinogen und/oder Proteoglykan basiert.

3. Verfahren nach Anspruch 1 oder 2, wobei das Implantat ein Herzklappenimplantat ist, das ganz oder teilweise eine Aortenklappe und/oder ein Herzbeutel eines Rinds, Schweins oder Schafs umfasst.

4. Verfahren nach einem der vorhergehenden Ansprüche, wobei in Schritt (A) eine Verbindung verwendet wird, die mindestens zwei Aldehydgruppen trägt.

5. Verfahren nach einem der vorhergehenden Ansprüche, wobei die Verbindung, die ein Borhydrid umfasst, die in Schritt (B) verwendet wird, ein Alkalimetallderivat ist.

6. Verfahren nach Anspruch 5, wobei die Verbindung, die ein Borhydrid umfasst, die in Schritt (B) verwendet wird, Natriumcyanoborhydrid ist.

7. Verfahren nach einem der vorhergehenden Ansprüche, wobei das Derivat, das eine Silangruppe trägt, das in Schritt (C) benutzt wird, eine elektronenziehende Gruppe umfasst, die direkt an das Siliciumatom gebunden ist, und ausgewählt ist aus den Halogenen, den Heteroarylgruppen, die 5 bis 15 Kohlenstoffatome und 2 oder 3 Heteroatome umfassen, die aus der Gruppe bestehend aus den Halogenen, den Pniktogenen und den Chalkogenen ausgewählt sind.

8. Verfahren nach Anspruch 7, wobei die elektronenziehende Gruppe, die in dem Derivat vorhanden ist, das eine Silangruppe trägt, das in Schritt (C) benutzt wird, ein Chloratom, ein Bromatom oder eine Imidazolgruppe ist.

9. Verfahren nach Anspruch 8, wobei das Derivat, das eine Silangruppe trägt, das in Schritt (C) verwendet wird, Trimethylsilylimidazol oder Chlortrimethylsilan ist.

10. Verfahren nach einem der vorhergehenden Ansprüche, das einen Zwischenschritt (A1) umfasst, der zwischen den Schritten (A) und (B) liegt, worin das Substrat, wie es am Ende von Schritt (A) erhalten wird, vor der Durchführung der Schritte (B) und (C) mit einer Verbindung behandelt wird, die mindestens zwei Aminfunktionen enthält.

11. Verfahren nach Anspruch 10, worin die Verbindung, die mindestens zwei Aminfunktionen trägt, ein Diamin mit der Formel NH₂-A-NH₂ ist, worin A für eine lineare oder verzweigte Kohlenwasserstoffkette steht, die 1 bis 20 Kohlenstoffatome trägt und die gegebenenfalls mit einem oder mehreren Heteroatomen substituiert sind, die aus der Gruppe bestehend aus den Halogenen, den Pniktogenen und den Chalkogenen ausgewählt sind.

12. Verfahren nach Anspruch 11, wobei die Verbindung, die mindestens zwei Aminfunktionen trägt, Poly(propylenglycol)bis(2-aminopropylether), Lysin, Spermin oder Putrescin ist.

13. Behandeltes Implantat, das auf Proteinen basiert, das am Ende des Verfahren zur Behandlung nach einem der vorhergehenden Ansprüche erhalten werden kann.

14. Implantat nach Anspruch 13, worin das Implantat im Wesentlichen frei von freien Aldehyd(-CHO)-Funktionen und Iminfunktionen ist.

15. Implantat nach Anspruch 13 oder 14, worin das Implantat ein Herzklappenimplantat ist.
